# EUROPEAN PATENT APPLICATION

(11) **EP 4 821 467 A2**
(43) Date of publication of application: **14.10.2026**
(21) Application number: 26201758.5
(22) Date of filing: 28.03.2024
(51) Int. Cl.: A61F 5/445

(54) **AN OSTOMY BODY FITMENT**

(30) Priority: 30.03.2023 US 202363493101 P; 12.07.2023 GB 202310676
(62) Document number(s) of the earlier application(s) in accordance with Art. 76 EPC: 24720999.2 / 4 687 786
(71) Applicant: ConvaTec Limited, Deeside, Flintshire CH5 2NU (GB)
(72) Inventor: Siebert, Jost, Deeside, CH5 2NU (GB)
(74) Representative: Wilson Gunn

(57) **Abstract**

An ostomy body fitment for forming a seal between an ostomy appliance and a stoma of an ostomate. The ostomy body fitment has an adhesive pad and a first removable release layer affixed to a first side of the adhesive pad. The adhesive pad has a stomal aperture formed therethrough. The first removable release layer comprises an image on a surface thereof. When the ostomy body fitment is in an unused state, the image is at least partially concealed by a part of the ostomy body fitment when viewed from a second side of the adhesive pad, opposite the first side. The part is moveable between a first position in the unused state to a second position in which the image is at least partially revealed through the stomal aperture when viewed from the second side of the adhesive pad.

## Description

### Technical Field of the Invention

The present invention provides an ostomy body fitment for forming a seal between an ostomy appliance and a stoma of an ostomate.

### Background to the Invention

Individuals that cannot pass waste naturally due to illness, injury or disease may require a colostomy, ileostomy, or urostomy. A colostomy or ileostomy involves attaching a section of the colon or small intestine respectively to an opening in the abdominal wall. A urostomy involves attaching a section of the urinary system to the abdominal wall in cases where drainage of urine through the bladder and urethra is not possible. In each case, a stoma for the excretion of waste from the body is formed at the opening that protrudes from the abdominal wall.

An ostomy appliance generally comprises a skin barrier or ostomy body fitment with a stomal aperture and a pouch for the collection of excreted waste. The ostomy body fitment of the ostomy appliance may be affixed by adhesive to the body of an individual around the stoma for the collection of excreted waste from the stoma into the pouch. An ostomy appliance may be provided as a single piece comprising both the ostomy body fitment and the pouch in a single pouch system. Alternatively, the ostomy appliance may be provided as a multi-piece (e.g., two piece) pouch system in which the ostomy body fitment and the ostomy pouch are separate components and may be attached together. For a multi-piece system, the ostomy body fitment may be first attached to the body of the ostomate around the stoma and the pouch may be attached to the ostomy body fitment to form the ostomy appliance for the collection of waste. Regardless of the system, it is important that an effective seal is formed between the stoma and the ostomy appliance to prevent leakage of excreted waste material.

Since ostomy appliances are often fitted by the patkient themselves, it is also important to provide a means for users to attach the ostomy appliance to their body easily, securely, and reliably, and without discomfort. Furthermore, since ostomy appliances are unsightly, it is important to try and ensure such devices are as discrete as possible.

Reference is made to EP1378219 which describes an ostomy appliance incorporating a shapeable adhesive which can be manipulated to form a custom shaped fit around a wearer's stoma.

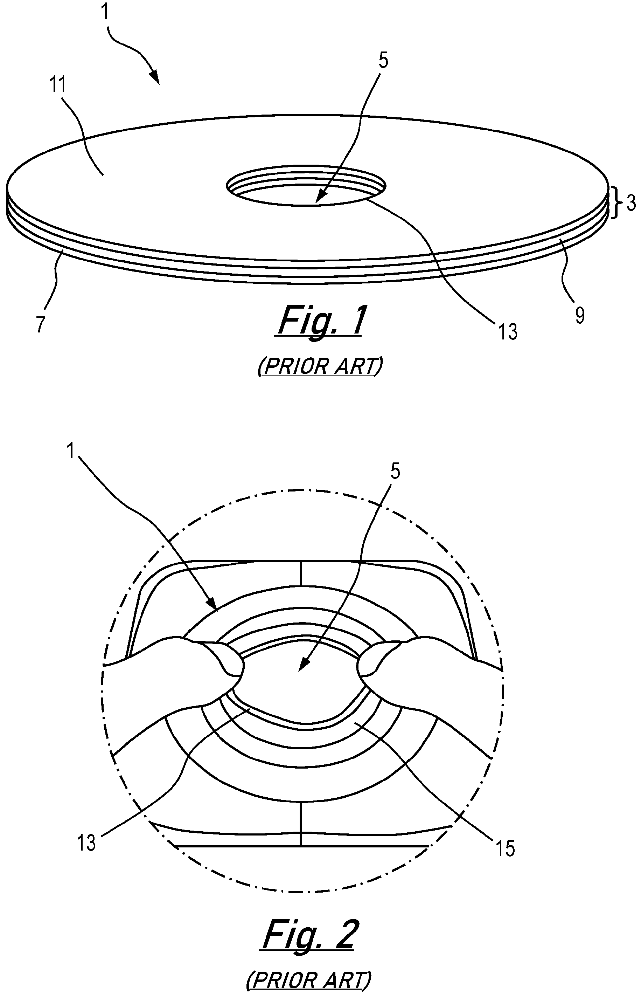

Reference is also made to Figures 1 to 3 which depict existing skin barriers or ostomy body fitments 1 for use in providing a seal between the user and an ostomy appliance comprising a tri-laminate structure 3 with a centrally located stomal aperture 5. The tri-laminate structure comprises a first, skin-facing, depicted lower, layer 7 comprising an adhesive for attaching the device to the skin of a patkient, a second, elastic mid-layer 9 with a degree of memory for rebounding and hugging the stoma, and a third, pouch facing, depicted upper layer 11 comprising an adhesive hydrocolloid material operable to swell and hug the stoma, when in use. A removable release layer or liner (not shown) may be attached to the skin-facing layer 7 and also to the pouch-facing layer 11 to protect the respective layers until installation of the device by a user.

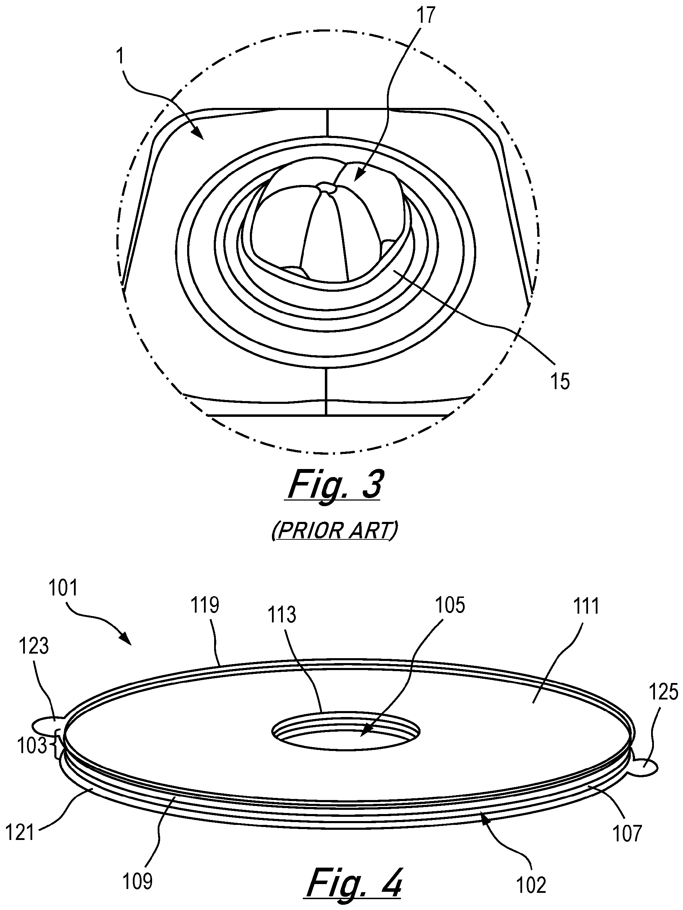

The ostomy body fitment 1 is intended to be installed according to a procedure comprising multiple, sequential operational steps. The operational steps include removing the release layer from the pouch-facing layer 11 and as shown in Figure 2, rolling the tri-laminate structure 3 from the inner edge 13 of the stomal aperture 5 outwardly and upwardly to form a raised collar or "turtleneck" 15. As shown in Figure 3 the device is affixed to the skin of the patkient such that the stoma 17 extends through the stomal aperture 5 of the body fitment 1 and the rolled collar 15 extends upwardly from the pouch-facing layer 11 and hugs the external wall of the stoma 17 with a snug fit. An ostomy pouch (not shown) may then be adhered to the ostomy body fitment 1, via the adhesive surface of the pouch-facing layer 11 such that a secure and reliable seal is formed between the stoma 17 and the resulting ostomy appliance.

One problem with existing ostomy body fitments as described above is that the multi-step application procedure is difficult for users to follow, even with accompanying instructions. Consequently, users frequently remove the wrong release layer at the wrong time and/or roll the device in the wrong direction. This can result in the device being incorrectly applied such that the collar extends inwardly toward the user and/or such that the wrong adhesive side is affixed to the skin of the user. The incorrect application of the ostomy body fitment may compromise the effectiveness of the seal between the ostomate and an ostomy appliance, and thereby give rise to undesirable leakage from the stoma, as well as discomfort.

The present invention seeks to provide an improved ostomy body fitment that at least mitigates the above problems.

### Summary of the Invention

The present invention provides an ostomy body fitment according to the appended claims.

The present disclosure provides an ostomy body fitment. The ostomy body fitment may be suitable for forming a seal between an ostomy appliance and a stoma of an ostomate. The ostomy body fitment may comprise an adhesive pad. A first removable release layer may be affixed to a first side of the adhesive pad. The adhesive pad may comprise a stomal aperture formed therethrough. The first removable release layer may comprise an image on a surface thereof. When the ostomy body fitment is in an unused state, the image may be at least partially concealed by a part of the ostomy body fitment. The image may be at least partially concealed by the part of the ostomy body fitment when viewed from a second side of the adhesive pad, opposite the first side. The part may be moveable between a first position in the unused state to a second position in which the image is at least partially revealed, e.g. through the stomal aperture, when viewed from the second side of the adhesive pad.

Accordingly, a first aspect of the invention provides an ostomy body fitment for forming a seal between an ostomy appliance and a stoma of an ostomate, the ostomy body fitment comprising an adhesive pad and a first removable release layer affixed to a first side of the adhesive pad, the adhesive pad comprising a stomal aperture formed therethrough, wherein the first removable release layer comprises an image on a surface thereof, wherein, in a unused state, the image is at least partially concealed by a part of the ostomy body fitment when viewed from a second side of the adhesive pad, opposite the first side, and wherein the part is moveable between a first position in the unused state to a second position in which the image is at least partially revealed through the stomal aperture when viewed from the second side of the adhesive pad.

Advantageously, the image can be concealed until it is desired to reveal the image to a user of the ostomy body fitment. This may be particularly useful where the image relates to an operational step of a series of operational steps in the correct application of the ostomy body fitment to the body of an ostomate. Where the operational step indicated by the image is preceded by one or more other operational steps, it is advantageous to conceal the image until the point at which the operational step should be carried out. This helps to ensure the user of the ostomy body fitment carries out each operational step in the correct sequence and, thereby, helps to ensure the user correctly installs the ostomy body fitment to the ostomate. At present it is considered that a barrier to the use of certain ostomy body fitments (in particular those that make up one part of a two-part ostomy pouch system and are intended to be adhered on one side to the user's skin and on the other side subsequently adhered to a pouch) is that users do not understand, or do not even read, the instructions that are provided in the packages in which body fitments are supplied. This can, for example, lead to the wrong side of the body fitment being adhered to the user's body, or the fitment being rolled in the wrong direction, so that it does not seal well to the user's body.

The part of the ostomy body fitment that at least partially conceals the image in the unused state may completely conceal the image in the unused state.

The part of the ostomy body fitment that at least partially conceals the image in the unused state may comprise a part of the adhesive pad. The adhesive pad may be deformable to expand the size of the stomal aperture. The adhesive pad and the first removable release layer may be relatively arranged such that the image is proximal to an edge of the stomal aperture (when in an unused state). The adhesive pad and the first removable release layer may be relatively arranged such that the image is revealed when the adhesive pad is manipulated to the second position (in which the size of the stomal aperture may be increased).

The image may be located on a region of the surface of the first removable release layer that lies between the boundary of the stomal aperture in an unexpanded state and the boundary of the stomal aperture in a maximum expanded state.

The adhesive pad may be deformable to expand the size of the stomal aperture and the image may be located on a region of the surface of the first removable release layer that lies between the boundary of the stomal aperture in an unexpanded state and the boundary of the stomal aperture in its maximum expanded state.

The image may be located on the surface of the first removable release layer within 10mm of the edge of the stomal aperture in the first position. The image may be arranged within 5mm, 4mm, 3mm or 2mm of the edge of the stomal aperture. The image may curve with the edge of the stomal aperture such that it extends at least partially around the stomal aperture.

The adhesive pad may be rollable (e.g., to increase the size of the stomal aperture). The image may be revealed when the adhesive pad is rolled outwards from the edge of the stomal aperture. The image may be located on a region of the surface of the first removable release layer that lies within the boundary of the stomal aperture defined by the adhesive pad in the maximum rolled state.

The ostomy body fitment may further comprise a second removeable release layer accessible from the second side of the adhesive pad. The part at least partially concealing the image in the unused state may comprise the second removable release layer i.e. the second removable release layer is a movable part of the ostomy body fitment. The second removable release layer may comprise an adhesive sticker.

Accordingly, one preferred embodiment of the invention provides an ostomy body fitment for forming a seal between an ostomy appliance and a stoma of an ostomate, the ostomy body fitment comprising an adhesive pad, a first removable release layer affixed to a first side of the adhesive pad, and a second removeable release layer accessible from the second side of the adhesive pad; the adhesive pad comprising a stomal aperture formed therethrough, wherein the first removable release layer comprises an image on a surface thereof, wherein, in an unused state, the image is at least partially concealed by a the second removable release layer when viewed from a second side of the adhesive pad, opposite the first side, and wherein the second removable release layer is moveable between a first position in the unused state to a second position in which the image is at least partially revealed through the stomal aperture when viewed from the second side of the adhesive pad.

The first removeable release layer may comprise a further image at least partially concealed by the second removable release layer in an unused state. The first removeable release layer and the second removeable release layer may be relatively arranged such that the image and/or the further image is at least partially revealed when the second removable release layer is removed from the rest of the ostomy body fitment.

The movement between a first position in the unused state to a second position in which the image is at least partially revealed through the stomal aperture when viewed from the second side of the adhesive pad may be removal of the second removable release layer.

Accordingly, another preferred embodiment of the invention provides an ostomy body fitment for forming a seal between an ostomy appliance and a stoma of an ostomate, the ostomy body fitment comprising an adhesive pad, a first removable release layer affixed to a first side of the adhesive pad, and a second removeable release layer accessible from the second side of the adhesive pad; the adhesive pad comprising a stomal aperture formed therethrough, wherein the first removable release layer comprises an image on a surface thereof, wherein, in an unused state, the image is at least partially concealed by a the second removable release layer when viewed from a second side of the adhesive pad, opposite the first side, and wherein the second removable release layer is moveable between a first position in the unused state to a second position in which the second removable release layer is removed from the rest of the ostomy body fitment and the image is at least partially revealed through the stomal aperture when viewed from the second side of the adhesive pad.

The second removeable release layer may comprise one or more of numbers, letters, pictorial representations, and patterns on a surface thereof and arranged to conceal the image at least partially and/or the further image in the unused state. The second removeable release layer may be opaque.

Alternatively, the second removable release layer may be translucent. The translucency of the second removable release layer may be such that features of the adhesive pad touching the release layer are visible through the second removable release layer. The translucency of the second removable release layer may be such that features separated from the second removable release layer for example by more than 0.1mm, 0.2mm, 0.5mm, 1mm are not visible through the second removable release layer. The first removable release layer may be separated from the second removable release layer by at least 0.1mm, 0.2mm, 0.5mm, 1mm or 2mm, for example by the thickness of the intervening adhesive pad. As such, there may be a gap of at least 0.1mm, 0.2mm, 0.5mm, 1mm or 2mm, between the first removable release layer and the second removable release layer in the region of the stomal aperture. Accordingly, the image and/or the further image may be concealed in the unused state because the translucency of the second removable release layer renders features on the first removable release layer invisible through the second removable release layer on account of the gap in the region of the stomal aperture.

The image and/or the further image may relate to an operational step in the use of the ostomy body fitment. The first removable release layer may be arranged relative to the part such that the image and/or the further image is at least partially revealed following completion of one or more preceding operational steps in the use of the ostomy body fitment that results in the movement of the part from the first position to the second position.

The image may comprise indicia. The image/indicia may comprise one or more of numbers, letters, pictorial representations, and patterns. The image may comprise text that proceeds in a forward direction when viewed from the second side of the adhesive pad. Hence, the text may be mirrored when viewed from the first side of the adhesive pad, opposite the second side.

The stomal aperture may be circular in the unused state. The stomal aperture may be formed through the centre of the adhesive pad. The adhesive pad may be disc shaped. The adhesive pad may be substantially planar.

The adhesive pad may comprise a convex or concave contour. The adhesive pad may comprise a tri-laminate structure. The tri-laminate structure may comprise a skin-facing layer, a pouch-facing layer, and a substrate layer between the skin-facing layer and the pouch-facing layer. The skin-facing layer and the pouch-facing layer may comprise adhesive. At least one of the skin-facing and pouch-facing layers may be mouldable. The adhesive pad may comprise one part of a two-part ostomy appliance pouch system. The adhesive pad may be releasably attachable to an ostomy pouch.

In a second aspect, the present invention provides a method of forming a seal between an ostomy appliance and a stoma of an ostomate comprising utilizing an ostomy body fitment according to the first aspect of the invention.

The method may comprise the step of moving part of the ostomy body fitment from a first position in the unused state to a second position in which the image is at least partially revealed through the stomal aperture when viewed from the second side of the adhesive pad. The method may comprise rolling part of the ostomy body fitment. In particular, the method may comprise rolling the adhesive pad to reveal the image.

The method may comprise the step of attaching a pouch of the ostomy appliance to the adhesive pad. The pouch may be attached to the adhesive pad after the adhesive pad is affixed to the body of the ostomate.

In a third aspect, the present invention provides an ostomy appliance comprising an ostomy body fitment according to the first aspect of the invention. The ostomy appliance may further comprise an ostomy pouch. The ostomy pouch and the ostomy body fitment may be releasably attachable.

In a fourth aspect, the present invention provides a method of manufacturing an ostomy body fitment comprising the steps of:
forming an adhesive pad defining a stomal aperture;
applying an image to a release layer; and
affixing the release layer to a first side of the adhesive pad such that the image is at least partially concealed by a moveable part of the ostomy body fitment in an unused state when viewed from a second side of the adhesive pad, opposite the first side;
wherein the moveable part is moveable between a first position in the unused state to a second position in which the image is at least partially revealed through the stomal aperture when viewed from the second side of the adhesive pad.

The step of applying the image to the release layer may comprise printing or painting the image.

The skilled person will appreciate that except where mutually exclusive, a feature described in relation to any one of the aspects, embodiments or examples described herein may be applied to any other aspect, embodiment, or example. Furthermore, except where mutually exclusive, any feature described herein may be applied to any aspect and/or combined with any other feature described herein.

### Brief Description of the Drawings

In order that the invention may be more clearly understood one or more embodiments thereof will now be described, by way of example only, with reference to the accompanying drawings, of which:
- Figure 1: shows a perspective view of a schematic representation of an existing ostomy body fitment;
- Figure 2: shows a perspective view of a render of an existing ostomy body fitment being manipulated by a user to shape a stomal aperture;
- Figure 3: shows the ostomy body fitment of Figure 2 when applied to the body of an ostomate such that the stoma of the ostomate extends through the stomal aperture;
- Figure 4: shows a perspective view of a schematic representation of an example ostomy body fitment in respect of which the present invention may be implemented;
- Figure 5: shows a plan view of an ostomy body fitment according to the present invention;
- Figure 6: shows a plan view of the ostomy body fitment shown in Figure 5 with a release liner removed;
- Figure 7: shows a plan view of the ostomy body fitment shown in Figure 6 in which part of the ostomy body fitment has been moved from a first position to a second position to increase the diameter of the stomal aperture and reveal an instructional image on a further release liner;
- Figure 8: shows an underside view of the ostomy body fitment shown in Figure 7 with a further release liner removed;
- Figure 9: shows a plan view of the ostomy body fitment shown in Figure 8;
- Figure 10: shows a plan view of the ostomy body fitment shown in Figure 9 affixed to the body of an ostomate such that the stoma of the ostomate extends through the stomal aperture of the ostomy body fitment;
- Figure 11: shows a plan view of the adhesive pad of the ostomy body fitment shown in Figure 10 with the last release liner removed;
- Figure 12: shows a plan view of the adhesive pad shown in Figure 11 affixed to the body of an ostomate and with part of a pouch of an ostomy appliance attached to the adhesive pad;
- Figure 13: shows a perspective view of a render of an ostomy body fitment according to the present invention housed within a sealed tray;
- Figure 14: shows a plan view of the tray and ostomy body fitment shown in Figure 13 with a foil liner removed from the tray to expose the ostomy body fitment seated within the tray;
- Figure 15: shows a plan view of an ostomy body fitment according to a second embodiment of the present invention;
- Figure 16: shows a plan view of the ostomy body fitment shown in Figure 15 with a release liner removed;
- Figure 17: shows a plan view of the ostomy body fitment shown in Figure 16 in which part of the ostomy body fitment has been moved from a first position to a second position to increase the diameter of the stomal aperture.

### Detailed Description of the Invention

In the following detailed description, numerous specific details are set forth in order to provide a thorough understanding of various embodiments and the inventive concept. However, those skilled in the art will understand that the present invention may be practiced without these specific details or with known equivalents of these specific details, that the present invention is not limited to the described embodiments, and that the present invention may be practiced in a variety of alternative embodiments. It will also be appreciated that well known methods, procedures, components, and systems may have not been described in detail.

In the following description, reference to "skin-facing" and "pouch-facing" should be taken to be in relation to the body of a user or "ostomate" of an ostomy body fitment, unless otherwise stated. For example, a skin-facing layer should be taken to be the layer intended to be affixed or attached to the skin of the ostomate, and a pouch-facing layer should be taken to be the layer intended to face away from the body of the ostomate when the skin-facing layer is affixed to the skin of the ostomate.

Figure 4 depicts an ostomy body fitment 101 of a two-piece ostomy pouch system for forming a seal between a stoma (not shown) and an ostomy pouch (not shown). The ostomy body fitment 101 comprises an adhesive pad 102 having a tri-laminate structure 103. The tri-laminate structure 103 defines a stomal aperture 105 with an inner edge 113 for the receipt of a stoma 117 of an ostomate. The stomal aperture 105 is generally circular in its initial, undeformed state so as to substantially correspond to the generally circular outer wall of a stoma. The stomal aperture 105 may have an initial diameter of between 10mm and 100mm, preferably between 20mm and 60mm. Preferably, the stomal aperture 105 is deformable from a first diameter to a second diameter, and preferably by approximately 10mm. In one form, the initial diameter of the stomal aperture 105 is generally about 20mm and deformable up to a diameter of about 30mm. In another form, the initial diameter of the stomal aperture 105 is generally about 30mm and deformable up to a diameter of about 40mm. In another form, the initial diameter of the stomal aperture 105 is generally about 40mm and deformable up to a diameter of about 50mm.

The tri-laminate structure 103 of the adhesive pad 102 comprises a skin-facing adhesive layer 107, a middle substrate layer 109, and a pouch-facing adhesive layer 111. The skin-facing adhesive layer 107 and/or pouch-facing adhesive layer 111 are made from a pliable and/or mouldable and/or shapeable adhesive. For example, the two adhesive layers 107, 111 may comprises a medical grade adhesive such as a hypoallergenic hydrocolloid. Examples of suitable adhesives are those produced by ConvaTec under the brand names Stomahesive^{®} and Durahesive^{®}. Such adhesives are generally tacky, putty-like, and substantially non-elastic, with low memory. The choice of adhesive may depend on the intended purpose of the ostomy appliance and its duration of application. For example, where the ostomy appliance comprises a closed bag or pouch intended for short wear or an open bag or pouch intended for short/medium wear, the skin facing adhesive layer 107 may comprise Stomahesive^{®} and the pouch facing side 111 may comprise Durahesive^{®}. Where the ostomy appliance is intended to be used by a patient with an ileostomy or urostomy for medium/long wear, both adhesive layers 107, 111 may comprise the more durable Durahesive^{®}. At least one and preferably both adhesive layers 107, 111 are translucent so that the substrate layer 109 is visible from either or both sides of the ostomy body fitment 101.

The skin-facing and pouch-facing adhesive layers 107, 111 are each between around 0.3mm and 1.5mm in thickness. The middle substrate layer 109 is between 0.2mm and 1.0mm and is generally thinner than the individual adhesive layers 107, 111. In the embodiment shown, the skin-facing adhesive layer 107 is 0.38mm in thickness, the substrate layer 109 is 0.2mm in thickness, and the pouch-facing adhesive layer is 1.14mm in thickness. The pair of adhesive layers 107, 111 and the substrate layer 109 have a substantially uniform thickness throughout and are substantially coextensive i.e., they each extend across substantially the same area. In the embodiment shown, the three layers 107, 109, 111 are substantially disc shaped and each has an outer diameter of approximately 100mm. The skilled person will appreciate that one or more of the three layers 107, 109, 111 may have a variable thickness across their respective diameters so as to create regions of different material characteristics e.g., having varying rigidity or surface shape.

Each adhesive layer 107, 111 is covered by a release layer 119, 121 in the form of a thin, circular sheet made from, or coated by, a suitable material such as silicone to facilitate removal of the release layer 119, 121 from the corresponding adhesive layer 107, 111. Each release layer 119, 121 extends beyond the outer boundaries of the adhesive layers 107, 111 and serves to protect the adhesive layers 107, 111 until the ostomy body fitment 101 is utilized by an ostomate. Each release layer 119, 121 comprises a small tab 123, 125 which protrudes from the outer edge of the corresponding release layer 119, 121 to allow the release layers 119, 121 to be easily grasped by a user of the ostomy body fitment 101 and removed from the corresponding adhesive layers 107, 111 when being applied to an ostomate. As further discussed below, the release layers 119, 121 provide a surface on which one or more images (not shown) may be formed.

As used herein, an "image" may comprise one or more of numbers, letters, pictorial representations, and patterns, and may be created by one or more coloured mediums that may have multiple colours or contrasting textures.

Whilst the ostomy body fitment shown in Figure 4 comprises a single release layer 119, 121 to protect a corresponding adhesive layer 107, 111, it will be appreciated that more than one release layer 119, 121 may be provided per adhesive layer 107, 111. For example, two or more release layers may be provided that individually partially cover different regions of a corresponding adhesive layer 107, 111 and collectively cover the entire surface of the adhesive layer 107, 111. Advantageously, different regions of an adhesive layer 107, 111 may be exposed at different times so that one or more operational steps may be performed on part of the ostomy body fitment 101 whilst protecting other parts until a subsequent operational step in the installation of the ostomy body fitment 101.

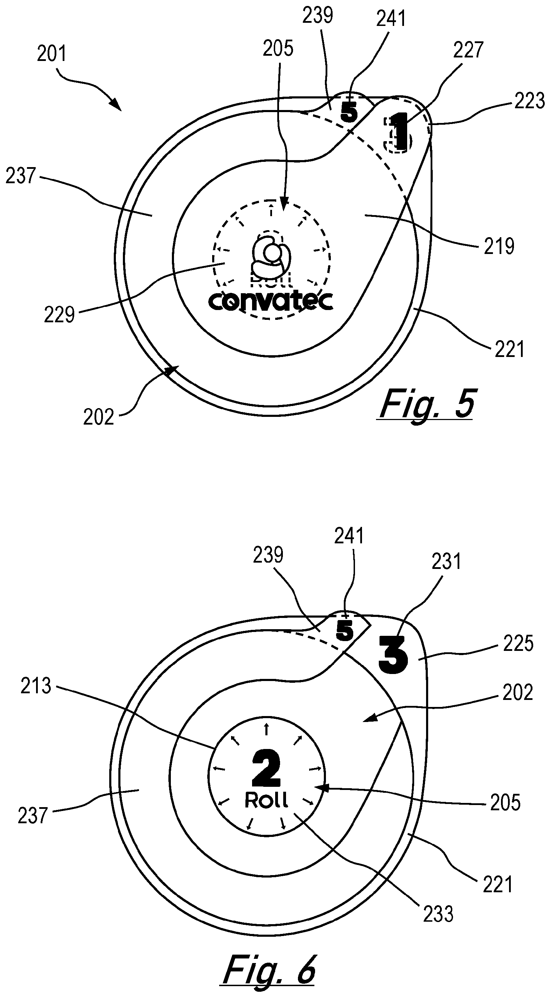

Referring to Figure 5, there is shown an ostomy body fitment 201 according to the present invention comprising an adhesive pad 202. The adhesive pad 202 of the ostomy body fitment 201 of Figure 5 is in many respects the same as that of Figure 4 and therefore like reference numerals (incremented by 100) are used for like features. Accordingly, the ostomy body fitment 201 of Figure 5 comprises a circular adhesive pad 202 with a tri-laminate structure 203 comprising a skin-facing adhesive layer 207, a middle substrate layer 209, and a pouch-facing adhesive layer 211 which together define a circular stomal aperture 205 through the centre of the adhesive pad 202. For the avoidance of doubt, the adhesive pad 202 may have the same dimensions and be made from the same materials as the adhesive pad 102 described above.

A first release layer 219 is affixed to the pouch-facing side of the adhesive pad 202. The first release layer 219 is shaped to cover the stomal aperture 205 and an inner region of the pouch-facing side of the adhesive pad 202. Accordingly, in the present embodiment, the first release layer 219 has a substantially circular main body that covers the entire stomal aperture 205 and an inner radial portion of the pouch-facing side of the adhesive pad 202. A portion of the first release layer 219 projects radially outwardly from the main body and beyond the outer boundary of the adhesive pad 202 to form a tab 223 for grasping. A first image 227 in the form of a number "1" is printed on the tab 223 to indicate to the user the order in which to remove the first release layer 219 from the adhesive pad 202.

The first release layer 219 further comprises a second image 229 at the centre of the main body portion in the form of a company logo e.g., a ConvaTec^{®} device mark. In addition to identifying the brand of the ostomy body fitment 201, the second image 229 is provided to obscure the stomal aperture 205 through the partially translucent first release layer 219 when viewed from the pouch-facing side of the ostomy body fitment 201. Of course, the second image 229 may comprise any type of image or even a block of solid colour that may serve to obscure the stomal aperture 205 and any underlying element that might otherwise be visible through the stomal aperture 205 when viewed from the pouch-facing side of the ostomy body fitment 201.

A second release layer 221 is affixed to the skin-facing side of the adhesive pad 202. The second release layer 221 has a greater surface area than the adhesive pad 202 such that the second release layer 221 covers the entirety of the skin-facing side of the adhesive pad 202 as well as the stomal aperture 205 and extends beyond the outer boundary of the adhesive pad 202. The second release layer 221 is substantially circular and additionally includes a tab 225 that extends radially outwardly from an outer edge of the second release layer 221 to facilitate grasping of the second release layer 221 by a user of the ostomy body fitment 201.

A third image 231 in the form of a number "3" is printed on the tab 225 of the second release layer 221. As above, the number is provided to indicate to a user of the ostomy body fitment 201 the order in which the second release layer 221 is intended to be removed from the adhesive pad 202 during an installation procedure comprising multiple sequential steps.

Referring to Figure 6, a fourth image 233 is printed on the second release layer 221 in a position at which the fourth image 233 is centred within the stomal aperture 205 when the adhesive pad 202 and the second release layer 221 are appropriately relatively arranged. In the present embodiment, the fourth image 233 comprises the number "2", the text "Roll" and a number of arrows extending radially outwardly from the centre of the second release layer 221 to indicate to the user of the ostomy body fitment 201 the direction in which to roll the adhesive pad 202 and the order in which to do so.

As shown in Figure 5, the first release layer 219 has a degree of opacity which serves to cover the fourth image 233 and at least partially obscure it when viewed from the pouch-facing side of the ostomy body fitment 201. The extent to which the fourth image 233 is obscured by the first release layer 219 may be further enhanced by the presence of the centrally located second image 229 which sits directly above the centre of the stomal aperture 205 when the adhesive pad 202 and the first and second release layers 219, 221 are appropriately relatively arranged. As discussed, advantageously, by obscuring one part of the ostomy body fitment 201 with another movable part of the ostomy body fitment 201, it is possible to defer when a written instruction relating to the utilization of the ostomy body fitment 201 is viewed, or at least clearly viewed, by a user and, therefore, possible to direct the user in the correct sequence of operational steps.

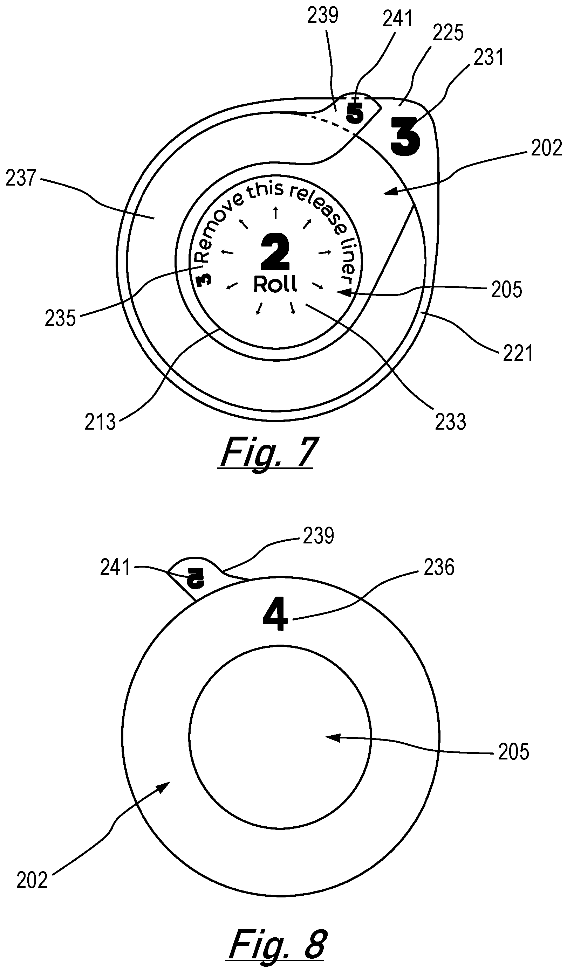

Referring to Figure 7, a fifth image 235 is printed on the second release layer 221 proximal to the edge 213 of the stomal aperture 205. The location of the fifth image 235 is such that it is concealed from view by the adhesive pad 202 when viewed from the pouch-facing side of the ostomy body fitment 201 and when the ostomy body fitment 201 is in an unused state and, hence, the adhesive pad 202 is in an undeformed state, as shown in both Figures 5 and 6. The location of the fifth image 235 proximal to the edge 213 of the stomal aperture 205 in the undeformed state is further such that it is revealed when a user manipulates the adhesive pad 202 to a deformed second position such as that shown in Figure 7 in which the diameter of the stomal aperture 205 has been increased, e.g. by rolling the adhesive pad 202 radially outwardly and upon itself. In the present embodiment, the fifth image 235 comprises the number "3" and the text "Remove this release liner", again to indicate to the user the procedural step to take and the order in which to do so. Preferably, the fifth image 235 is located within the maximum distance of deformation of the stomal aperture 205 from its initial state e.g., 5mm.

As above by obscuring the fifth image 235 with another movable part of the ostomy body fitment 201, it is possible to defer when the fifth image 235 is revealed to the user of the ostomy body fitment. For example, where the fifth image 235 comprises a visual instruction relating to the utilization of the ostomy body fitment 201, viewing of the fifth image 235 and, hence, the implementation of the corresponding visual instruction may be deferred until each preceding operational step has been completed. In this case, the operational step immediately preceding the operational step indicated by the fifth image 235 may involve moving the part of the ostomy body fitment 201 obscuring or concealing the fifth image 235 to a second position in which the fifth image 235 is revealed to the user.

Referring to Figure 8, a sixth image 236 in the form of the number "4" is printed on a skin-facing surface of the ostomy body fitment 201. In the present embodiment, the sixth image 236 is printed on the skin-facing surface of the mid-layer of the adhesive pad 202 beneath the skin-facing adhesive layer 207. Since the skin-facing adhesive layer 207 is translucent, the sixth image 236 is visible to the user when viewed from the skin-facing side of the ostomy pouch. The number depicted by the image indicates to the user the correct procedural step at which to apply the adhesive pad 202 to the skin of an ostomate.

As shown in Figures 5 to 7, a third release layer 237 having an arcuate form is affixed to the pouch-facing side of the adhesive pad 202 and extends around and covers the majority of the outer circumferential region of the pouch-facing side of the adhesive pad 202. The shape of the third release layer 237 is selected to complement the shape of the first release layer 219 so that they collectively cover the entire pouch-facing side of the adhesive pad 202 and the stomal aperture 205. One end of the third release layer 237 extends beyond the outer boundary of the adhesive pad 202 to form a tab 239 for grasping by a user. As can be seen, a seventh image 241 in the form of the number "5" is printed on the tab 239 to indicate to the user the order in which to remove the third release layer 237 when utilizing the ostomy body fitment 201.

As shown in Figures 5 to 8, each of the first to seventh images 227, 229, 231, 233, 235, 236, 241 are printed such that they proceed in the correct direction when viewed from the pouch-facing side of the ostomy body fitment 201. Accordingly, the number "3" or the text "Roll", for example, are mirrored when viewed from the skin-facing side of the ostomy body fitment 201. This may help to indicate to a user of the ostomy body fitment 201 the correct side from which to carry out the indicated steps and, hence, ensure that the user rolls the adhesive pad 202 in the correct direction and affixes the correct side of the adhesive pad 202 to the body of the ostomate.

It will be appreciated that any number of images may be applied to the release layers 219, 221, 237 as desired, subject to the available space, so as to provide visual instructions to a user in the correct utilization of the ostomy body fitment 201. It will also be appreciated that one or more images may be applied to the adhesive pad 202 itself, for example the substrate layer, to further compliment the instructions in the use of the ostomy body fitment 201. Accordingly, one or more images may be provided on the adhesive pad 202 and one or more images may be provided on the release layers 219, 221, 237 and each may relate to a sequence of instructions in the correct utilization of the ostomy body fitment.

Each of the release layers 219, 221, 237 is made from a suitable material such as silicone to facilitate removal of the release layer 219, 221, 237 from the corresponding adhesive layer. Each of the release layers 219, 221, 237 is further made from or coated in a material that facilitates printing or painting an image on a surface thereof.

Whilst the above embodiments describe and depict a disc shaped adhesive pad it will be appreciated that the adhesive pad may have a different shape such as square, star, rectangle, or oval, provided there is a surface through which a stomal aperture of appropriate size may be defined and expanded, and also provided there is sufficient surface area to ensure good contact with the body of an ostomate. Furthermore, whilst the above embodiments describe and depict an adhesive pad that is substantially planar, it will be appreciated that part or all of the adhesive pad may comprise a convex or concave contour. This may be desirable when used on an ostomate with a retracted stoma or a stoma that does not sufficiently protrude from the abdominal wall to extend through the stomal aperture of a planar ostomy body fitment. The convexity or concavity of the ostomy body fitment may serve to push against the abdominal wall of an ostomate and urge the stoma into a more protruded position for the capture of the stomal aperture. In such an arrangement clarity to the user in terms of which side to apply to the skin is even more important.

As mentioned, the ostomy body fitment 201 is preferably installed according to a process comprising multiple sequential steps. Referring to Figures 5 and 6, in a first step, the first release layer 219 is removed from the pouch-facing side of the adhesive pad 202 to expose the stomal aperture 205 and at least an inner region of the pouch-facing side of the adhesive pad 202 so that the edge 213 of the stomal aperture 205 can be accessed by the user. As shown in Figure 6, removing the first release layer 219 exposes the underlying fourth image 233 of the second release layer 221 which comprises instructions to implement the second step.

In accordance with the indicated second step, as shown between Figures 6 and 7, a user urges the inner edge 213 of the stomal aperture 205 upwardly and outwardly so as to roll the adhesive pad 202 upon itself along the pouch-facing surface of the adhesive pad 202. This has the effect of forming a raised collar extending around the circumference of the stomal aperture 205. The deforming and/or rolling action allows the user to increase the diameter of the stomal aperture 205 so as to approximately correspond to the diameter of the target stoma 217. As shown in Figure 7, increasing the diameter of the stomal aperture 205 serves to reveal the fifth image 235 and, hence, the visual instruction relating to the next operational step in the installation sequence.

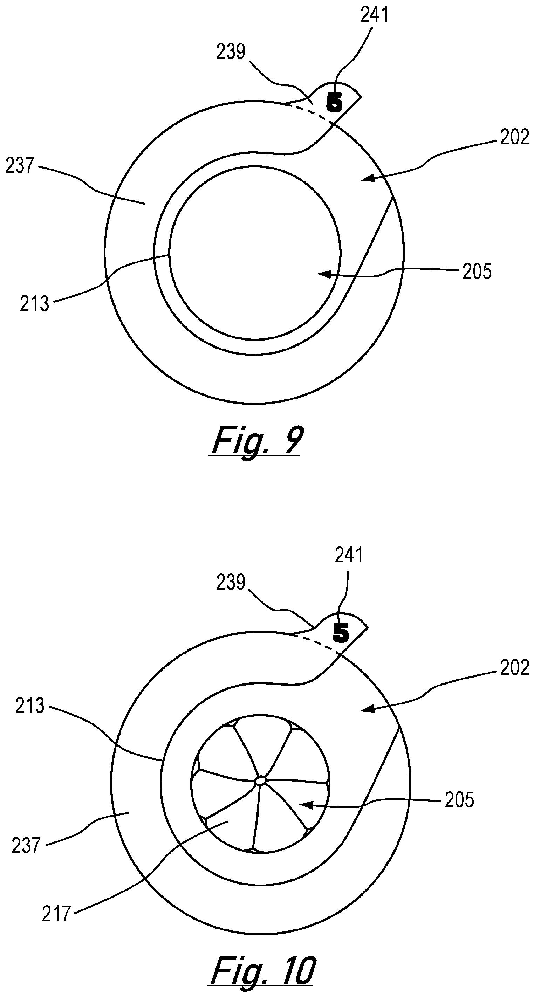

When the user has achieved a stomal aperture diameter that corresponds to or is slightly greater than the diameter of the target stoma 217, in accordance with the visual instruction of the fifth image 235, in a third step, the user removes the second release layer 221 from the skin-facing side of the adhesive pad 202 so as to completely open the stomal aperture 205, as shown in Figures 8 and 9.

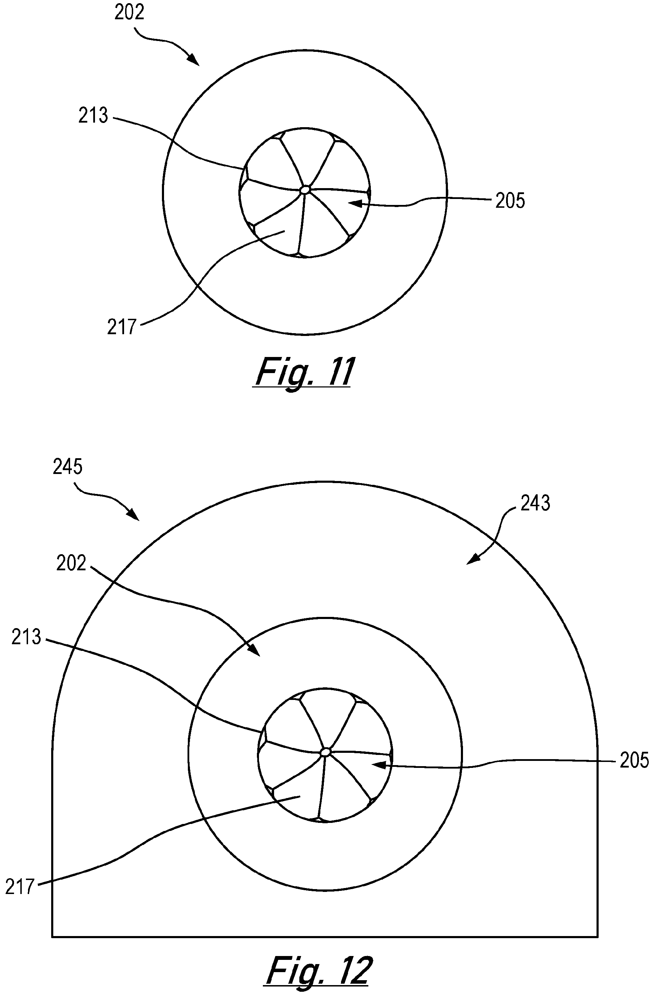

Referring to Figures 8 and 10, as indicated by the sixth image 236 visible from the skin-facing side of the adhesive pad 202, in a fourth step, the user affixes the adhesive pad 202 to the skin of the ostomate such that the stoma 217 extends through the stomal aperture 205. The third release layer 237 provides a surface to which the user may apply pressure when affixing the adhesive pad 202 to the ostomate without adversely affecting the pouch-facing adhesive layer of the adhesive pad 202. As shown between Figures 10 and 11, in a fifth step, the third release layer 237 is removed from the adhesive pad 202 so that only the adhesive pad 202 remains affixed to the body of the ostomate. In this position, the adhesive pad 202 encircles the stoma 217 and the collar formed in a preceding step extends upwardly along the outer wall of the stoma 217 away from the body of the ostomate. Due to the degree of memory of the substrate layer, the adhesive pad 202 rebounds such that the stomal aperture 205 decreases in diameter and the collar hugs the stoma 217 with a snug fit.

Referring to Figure 12, in a fifth and final step, the user affixes an ostomy pouch 243 to the adhesive pad 202 so as to form an ostomy appliance 245. Accordingly, a seal is formed between the ostomy appliance 245 and the stoma 217 such that waste is excreted into the pouch 243 from the stoma 217 without leakage.

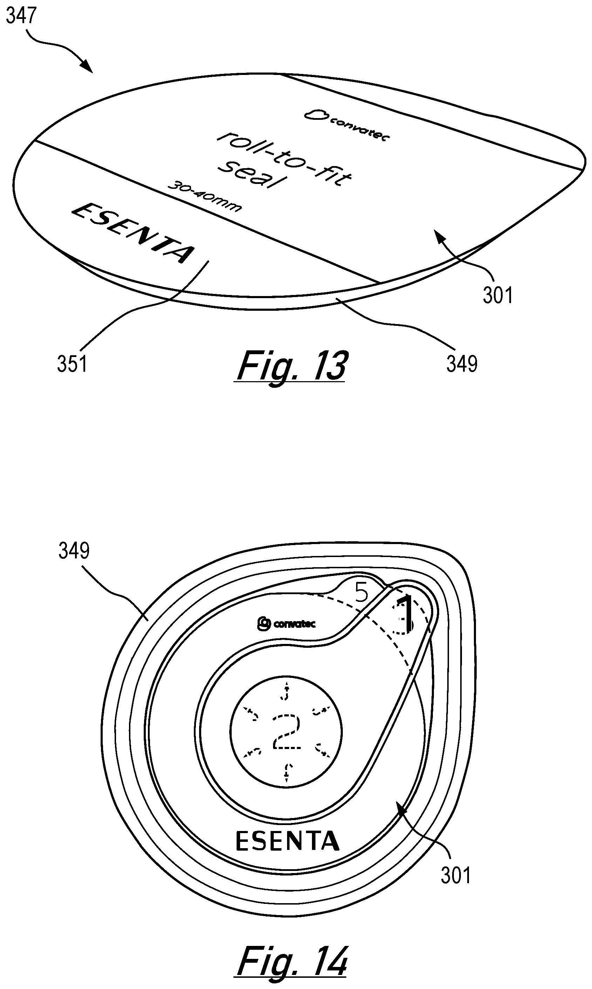

As shown in Figure 13 and 14, an ostomy body fitment 301 according to the present invention may be housed within a sealed tray 347. The ostomy body fitment 301 may be substantially the same as the ostomy body fitment 201 described above. The sealed tray 347 may comprise a tray base 349 made from plastics material which is shaped to receive and support the ostomy body fitment 301 in a substantially horizontal position. A foil liner 351 is provided to extend across an open face of the tray base 349 and thereby seal the ostomy body fitment 301 within the tray base 349. Accordingly, to access the ostomy body fitment 301, a user may remove the foil liner 351 from the tray base 349. The tray 347 provides a protective, sealed environment for the ostomy body fitment 301 to facilitate the transportation, storage, and sale of the ostomy body fitment 301.

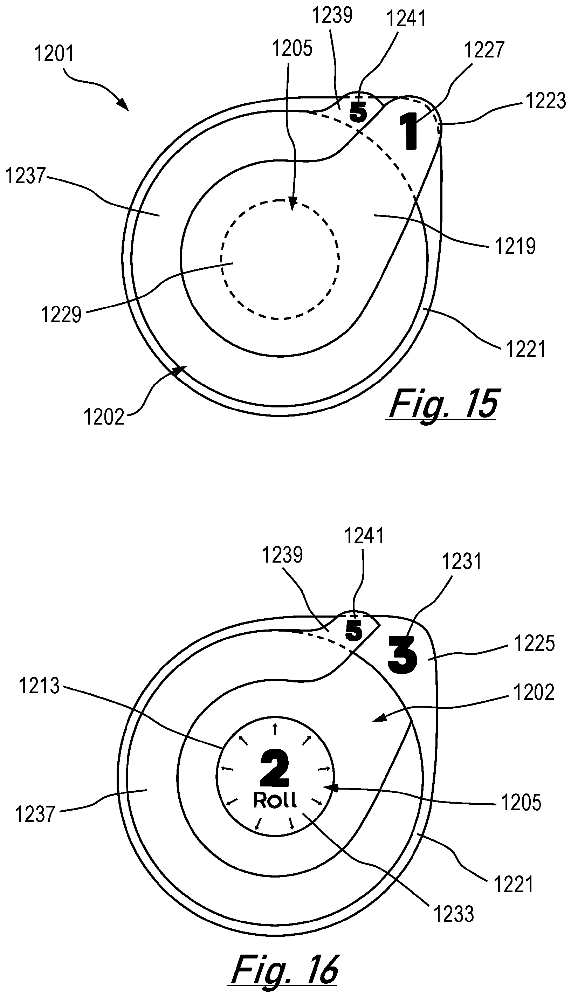

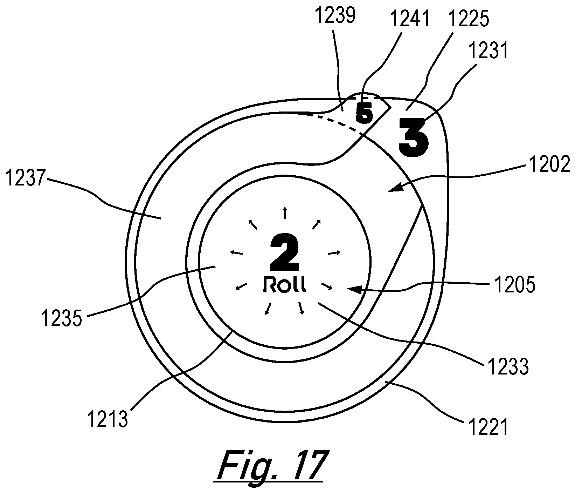

Figures 15 to 17 demonstrate an alternative embodiment of the invention, which shares many of the features of the first embodiment of the invention (of figures 5-12). Accordingly this description focusses on the differences and uses the same numbering system, incremented by 1000.

The first major difference in the second embodiment is in relation to the translucency of the first release layer 1219 and the printing thereon. In particular, the first release layer 219 of the first embodiment has a sufficient opacity to partially conceal the printing fourth image 233, with further partial concealing achieved by the second image 229 provided thereon. On the other hand, the material of the first release layer 1219 of this embodiment has no image thereon, but is completely blank in the region covering the stomal aperture 1229. However, its translucency is such that only the adhesive pad 1202 can be seen through the first release layer, because it is touching the first release layer 1219. The image 1233 on the second layer 1221 is completely concealed, because it is separated from the first release layer 1219 by an air gap equal to the thickness of the adhesive pad 1202 (i.e. about 1mm to 2mm).

The second major difference in the second embodiment is again concerned with the absence of a feature. In this embodiment, unlike the third embodiment, rolling up the adhesive pad 1202 does not reveal a further image 235, rather there is blank space 1235 in the region adjacent to the raised collar formed by rolling up the inner edge of the adhesive pad. As such, in this embodiment, the user must rely on the third image 1231 in the form of a number "3" is printed on the tab 1225 of the second release layer 1221 to instruct them to remove the second release liner 1225 after forming the collar.

Thus, whereas the first embodiment has two parts which are moveable between a first position in the unused state to a second position in which the image is at least partially revealed through the stomal aperture when viewed from the second side of the adhesive pad (namely the first release liner 219 and the rollable part of the adhesive pad 202), in the second embodiment there is only one part which is moveable between a first position in the unused state to a second position in which the image is at least partially revealed through the stomal aperture when viewed from the second side of the adhesive pad, namely the first layer 1219, which is movable from the first (unused) position in which the image 1221 is completely concealed to a second position (when it is removed), in which the image 1221 is visible.

Of course, the second embodiment is used in exactly the same manner as the first embodiment, as outlined above.

The one or more embodiments are described above by way of example only. Many variations are possible without departing from the scope of protection afforded by the appended claims.

Certain definitions of the invention are set out in the following clauses:

### CLAUSES

1. An ostomy body fitment for forming a seal between an ostomy appliance and a stoma of an ostomate, the ostomy body fitment comprising an adhesive pad and a first removable release layer affixed to a first side of the adhesive pad, the adhesive pad comprising a stomal aperture formed therethrough, wherein the first removable release layer comprises an image on a surface thereof, wherein, in an unused state, the image is at least partially concealed by a part of the ostomy body fitment when viewed from a second side of the adhesive pad, opposite the first side, and wherein the part is moveable between a first position in the unused state to a second position in which the image is at least partially revealed through the stomal aperture when viewed from the second side of the adhesive pad.
2. The ostomy body fitment as claimed in clause 1, wherein the part of the ostomy body fitment at least partially concealing the image in the unused state comprises a part of the adhesive pad.
3. The ostomy body fitment as claimed in clause 2, wherein the adhesive pad is deformable to the second position in which the size of the stomal aperture is increased, and wherein the adhesive pad and the first removable release layer are relatively arranged such that the image is proximal to an edge of the stomal aperture when in an unused state so that the image is revealed when part of the adhesive pad is deformed to the second position.
4. The ostomy body fitment as claimed in clause 3, wherein the image is located on a region of the surface of the first removable release layer that lies between the boundary of the stomal aperture in an unexpanded state and the boundary of the stomal aperture in a maximum expanded state.
5. The ostomy body fitment as claimed in any of clauses 2 to 4, wherein the part of the adhesive pad is rollable.
6. The ostomy body fitment of clause 5, wherein the image is located on a region of the surface of the first removable release layer that lies within the boundary of the stomal aperture defined by the adhesive pad in the maximum rolled state.
7. The ostomy body fitment of clause 3 or clause 4, wherein the image is located on the surface of the first removable release layer within 10mm of the edge of the stomal aperture in the first position.
8. The ostomy body fitment as claimed in any one of clause 3 to 7, wherein the image curves with the edge of the stomal aperture such that it extends at least partially around the stomal aperture.
9. The ostomy body fitment as claimed in clause 1 further comprising a second removeable release layer accessible from the second side of the adhesive pad; wherein the part at least partially concealing the image in the unused state comprises the second removable release layer.
10. The ostomy body fitment as claimed in clause 9 wherein the first removeable release layer and the second removeable release layer are relatively arranged such that the image is at least partially revealed when the second removable release layer is removed from the rest of the ostomy body fitment.
11. The ostomy body fitment as claimed in clause 9 or 10 wherein the second removable release layer is translucent.
12. The ostomy body fitment as claimed in clause 11 wherein the translucency of the second removable release layer is such that features separated from the second removable release layer by more than 1mm are not visible through the second removable release layer.
13. The ostomy body fitment according to clause 12 wherein the first removable release layer is separated from the second removable release layer by at least 1mm such that there is a gap of at least 1mm between the first removable release layer and the second removable release layer in the region of the stomal aperture, whereby the image is concealed in the unused state because the translucency of the second removable release layer renders features on the first removable release layer invisible through the second removable release layer on account of the gap in the region of the stomal aperture.
14. The ostomy body fitment as claimed in clause 9 or 10, wherein the second removeable release layer is opaque.
15. The ostomy body fitment as claimed in any of clauses 2 to 8 further comprising a second removeable release layer accessible from the second side of the adhesive pad, wherein the first removeable release layer comprises a further image at least partially concealed by the second removable release layer in an unused state, and wherein the first removeable release layer and the second removeable release layer are relatively arranged such that the further image is at least partially revealed when the second removable release layer is removed from the rest of the ostomy body fitment.
16. The ostomy body fitment as claimed in any one of clauses 9 to 15, wherein the second removeable release layer comprises one or more of numbers, letters, pictorial representations, and patterns on a surface thereof and arranged to conceal the image at least partially and/or the further image in the unused state.
17. The ostomy body fitment as claimed in any preceding clause, wherein the image relates to an operational step in the use of the ostomy body fitment, and wherein the first removable release layer is arranged relative to the part such that the image is at least partially revealed following completion of one or more preceding operational steps in the use of the ostomy body fitment that results in the movement of the part from the first position to the second position.
18. The ostomy body fitment as claimed in any preceding clause, wherein the image comprises one or more of numbers, letters, pictorial representations, and patterns.
19. The ostomy body fitment as claimed in any preceding clause, wherein the image comprises text that proceeds in a forward direction when viewed from the second side of the adhesive pad.
20. The ostomy body fitment as claimed in any preceding clause, wherein the stomal aperture is circular in the unused state.
21. The ostomy body fitment as claimed in any preceding clause, wherein the stomal aperture is formed through the centre of the adhesive pad.
22. The ostomy body fitment as claimed in any preceding clause, wherein the adhesive pad is disc shaped.
23. The ostomy body fitment as claimed in any preceding clause, wherein the adhesive pad is substantially planar.
24. The ostomy body fitment as claimed in any one of clauses 1 to 22, wherein the adhesive pad comprises a convex contour.
25. The e ostomy body fitment as claimed in any one of the preceding clauses wherein the part of the ostomy body fitment that at least partially conceals the image in the unused state completely conceals the image in the unused state.
26. An ostomy appliance comprising an ostomy body fitment as claimed in any one of clauses 1 to 25.

A method of manufacturing an ostomy body fitment comprising the steps of forming an adhesive pad defining a stomal aperture, applying an image to a release layer, and affixing the release layer to a first side of the adhesive pad such that the image is at least partially concealed by a moveable part of the ostomy body fitment in an unused state when viewed from a second side of the adhesive pad, opposite the first side, wherein the moveable part is moveable between a first position in the unused state to a second position in which the image is at least partially revealed through the stomal aperture when viewed from the second side of the adhesive pad.

## Claims

1. An ostomy body fitment for forming a seal between an ostomy appliance and a stoma of an ostomate, the ostomy body fitment comprising an adhesive pad; a first removable release layer affixed to a first side of the adhesive pad and a second removeable release layer accessible from the second side of the adhesive pad, the adhesive pad comprising a stomal aperture formed therethrough, wherein the first removable release layer comprises an image on a surface thereof, wherein, in an unused state, the image is at least partially concealed by the second removeable release layer when viewed from a second side of the adhesive pad, opposite the first side, and wherein the second removeable release layer is moveable between a first position in the unused state to a second position in which the image is at least partially revealed through the stomal aperture when viewed from the second side of the adhesive pad.

2. The ostomy body fitment as claimed in claim 1 wherein the first removeable release layer and the second removeable release layer are relatively arranged such that the image is at least partially revealed when the second removable release layer is removed from the rest of the ostomy body fitment.

3. The ostomy body fitment as claimed in claim 1 or 2 wherein the second removable release layer is translucent.

4. The ostomy body fitment as claimed in claim 3 wherein the translucency of the second removable release layer is such that features separated from the second removable release layer by more than 1mm are not visible through the second removable release layer.

5. The ostomy body fitment according to claim 4 wherein the first removable release layer is separated from the second removable release layer by at least 1mm such that there is a gap of at least 1mm between the first removable release layer and the second removable release layer in the region of the stomal aperture, whereby the image is concealed in the unused state because the translucency of the second removable release layer renders features on the first removable release layer invisible through the second removable release layer on account of the gap in the region of the stomal aperture.

6. The ostomy body fitment as claimed in claim 1 or 2, wherein the second removeable release layer is opaque.

7. The ostomy body fitment as claimed in any preceding claim, wherein the second removeable release layer comprises one or more of numbers, letters, pictorial representations, and patterns on a surface thereof and arranged to conceal the image at least partially in the unused state.

8. The ostomy body fitment as claimed in any preceding claim, wherein the image relates to an operational step in the use of the ostomy body fitment, and wherein the first removable release layer is arranged relative to the part such that the image is at least partially revealed following completion of one or more preceding operational steps in the use of the ostomy body fitment that results in the movement of the part from the first position to the second position.

9. The ostomy body fitment as claimed in any preceding claim, wherein the image comprises one or more of numbers, letters, pictorial representations, and patterns.

10. The ostomy body fitment as claimed in any preceding claim, wherein the image comprises text that proceeds in a forward direction when viewed from the second side of the adhesive pad.

11. The ostomy body fitment as claimed in any preceding claim, wherein the stomal aperture is circular in the unused state and the stomal aperture is formed through the centre of the adhesive pad.

12. The ostomy body fitment as claimed in any preceding claim, wherein the adhesive pad is disc shaped and is substantially planar or comprises a convex contour.

13. The ostomy body fitment as claimed in any one of the preceding claims wherein the second removeable release layer completely conceals the image in the unused state.

14. An ostomy appliance comprising an ostomy body fitment as claimed in any one of claims 1 to 13.

15. A method of manufacturing an ostomy body fitment comprising the steps of forming an adhesive pad defining a stomal aperture, applying an image to a first removeable release layer, and affixing the first removeable release layer to a first side of the adhesive pad such that the image is at least partially concealed by a second removeable release layer in an unused state when viewed from a second side of the adhesive pad, opposite the first side, wherein the second removeable release layer is moveable between a first position in the unused state to a second position in which the image is at least partially revealed through the stomal aperture when viewed from the second side of the adhesive pad.
